# EUROPEAN PATENT SPECIFICATION

(11) **EP 3 010 442 B1**
(45) Date of publication and mention of the grant of the patent: **07.12.2016**
(21) Application number: 14737082.9
(22) Date of filing: 05.06.2014
(51) Int. Cl.: A61F 2/01, A61F 2/24

(54) **PERCUTANEOUS EMBOLI PROTECTION SLEEVE**
PERKUTANE EMBOLIESCHUTZHÜLSE
MANCHON PERCUTANÉ DE PROTECTION CONTRE L'EMBOLIE

(30) Priority: 16.06.2013 US 201361835596 P
(43) Date of publication of application: 27.04.2016
(73) Proprietor: Pi-Cardia Ltd., 76706 Rehovot (IL)
(72) Inventor: GOLAN, Erez, 76304 Rehovot (IL)
(74) Representative: White, Duncan Rohan
(86) International application number: PCT/US2014/040991
(87) International publication number: WO 2014/204648

(56) References cited:
- WO-A1-03/075997
- WO-A2-03/077799
- US-A1- 2004 153 094
- US-A1- 2012 179 033

## Description

### FIELD OF THE INVENTION

The present invention generally relates to capturing embolic or other materials, such as during cardiovascular surgery or catheterization, and particularly to a percutaneous protection sleeve, which can slide over a catheter and the like.

### BACKGROUND OF THE INVENTION

PCT Patent Application PCT/US2013/035346, filed April 5, 2013, to the same inventor and current assignee, describes devices and methods for improving the safety and efficacy of percutaneous treatment of vasculature and devices introduced into the vasculature, such as the aortic valve. In one embodiment, there is a guidance and protection sleeve that can be positioned in the aorta or other body lumen. The guidance and protection sleeve can be used to accurately position a catheter (or new valve) for deployment by guiding the catheter (or the new valve) into the center of the native valve, without scraping the potentially calcified or atheromateous aortic wall. In another embodiment, the invention describes a percutaneous sleeve that slides over a catheter.

In some embodiments, the protection sleeve can create an enclosed (or partly enclosed) region above the native valve leaflets (above or below the coronary ostia), in order to capture or divert away from the cerebral vasculature any embolic debris that might be created during impact, decalcification, dilation (such as by balloon valvuloplasty) or implantation of the new valve.

In some embodiments, the device can be used for assisting the delivery of catheters through blood vessels by reducing the risk of scraping or perforating the blood vessel walls during catheter delivery and retrieval. The device can transform its shape after it is delivered, and before use in catheter delivery. For example, the device expands after delivery to the site before catheter delivery. Catheters may be delivered through a lumen in the device. The device can assist relative positioning of the catheter vis-à-vis the anatomy. The device can assist in centering the catheter in the aorta. The device can aid in the capture or diversion of embolic debris created during treatment.

US-A-2012/179033 discloses a device comprising:a filter having a proximal end and inserted within an outer sheath. A distal end is joined to an intermediate tube, wherein said outer sheath slides over said intermediate tube. A catheter passes through a lumen of said intermediate tube. A distal portion of said catheter comprises a medical device. Relative sliding movement of said outer sheath and intermediate tube with respect to each other either causes contraction or expansion of said filter.

### SUMMARY OF THE INVENTION

The present invention seeks to provide further improvements to the devices and methods of PCT Patent Application PCT/US2013/035346, as is described more in detail hereinbelow.

The term "catheter" as used herein, encompasses any percutaneous device, such as but not limited to, a catheter, cannula, guidewire, stent, certain transcatheter aortic valve implantation (TAVI) devices, and others. The catheter may be a treatment catheter, diagnostic catheter, imaging catheter, etc.

There is provided in accordance with one embodiment of the present invention, a device including a protection sleeve which has a proximal end joined to a first shaft and a distal end preferably joined to a second shaft, wherein the first shaft preferably slides over the second shaft, and a catheter that preferably passes through a lumen of the second shaft, wherein a distal portion of the catheter includes a medical device; and wherein relative sliding movement of the first and preferably second shafts with respect to each other either causes contraction or expansion of the protection sleeve. The relative movement of both shafts with respect to the catheter may control the axial position of the protection sleeve with respect to the catheter, so if the catheter is fixed vis-à-vis the anatomy, the protection sleeve may be positioned relative to the catheter, and therefore relative to the anatomy as well. Alternatively, only one shaft can be used (either proximal or distal), and the catheter slides through a lumen in the shaft, wherein one end of the protection sleeve is connected to the catheter and the other end is connected to the shaft.

### BRIEF DESCRIPTION OF THE DRAWINGS

The present invention will be understood and appreciated more fully from the following detailed description, taken in conjunction with the drawings in which:

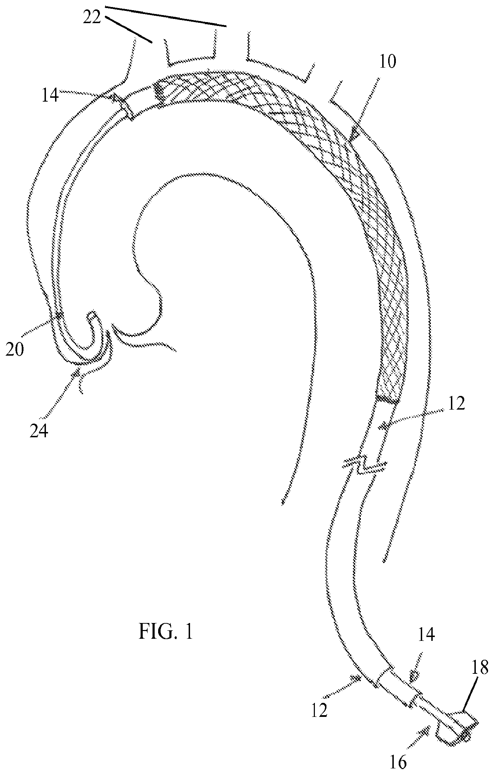

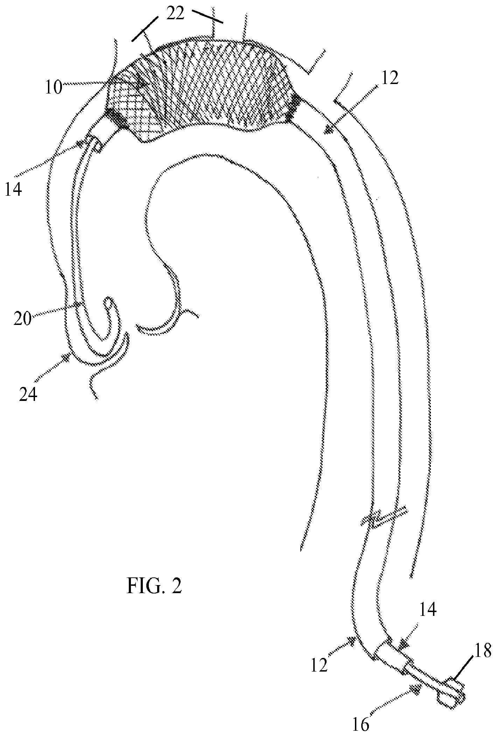

Figs. 1 and 2 are simplified illustrations of a protection sleeve, in respective collapsed and expanded orientations, deployed and positioned to protect carotid takeoffs from emboli entering therein, in accordance with a non-limiting embodiment of the present invention.

### DETAILED DESCRIPTION OF EMBODIMENTS

Reference is now made to Figs. 1 and 2, which illustrate a protection sleeve 10, constructed and operative in accordance with a non-limiting embodiment of the present invention. Protection sleeve 10 may be constructed from a medically safe plastic, metal or other material, such as without limitation, stainless steel, shape memory alloy, titanium alloy, polymers, etc.

In one preferred construction, protection sleeve 10 is formed of braided wire or mesh. Constructing protection sleeve 10 with braided wire technology has the advantage that the "pitch" of each wire can be relatively large when in the contracted state and then relatively small when in the fully expanded state. In the contracted state, the pores created by the braided wires have elongated "diamond" shapes (long and narrow) and therefore add minimal thickness to the catheter. In the expanded state, the pores have transverse "diamond" shapes (short and wide), that is, a very small pore size, which is advantageous for embolic protection. Thus, using braided wire technology, a relatively low profile (but long) sleeve can transform into a large expanded diameter sleeve with very small pore size.

A proximal end of protection sleeve 10 is joined to a first (e.g., proximal) shaft 12 and a distal end of protection sleeve 10 is joined to a second (e.g., distal) shaft 14. Joining may be accomplished by any suitable means, such as but not limited to, adhesive bonding, thermal bonding, braiding, and many others. First shaft 12 slides over second shaft 14. Both shafts may be delivered over a catheter 16, which in the illustrated embodiment is a pigtail catheter having a proximal portion 18 and a distal portion 20. The distal portion 20 includes a medical device, preferably a heart valve treatment device, such as a pigtail 20. Pigtail catheters may be useful in marking the valve plane (level of the sinuses), measuring arterial or cardiac blood pressures, or delivering fluid to the site, such as contrast material, for imaging the vasculature. In other words, catheter 16 passes through the lumen of the second shaft 14 and second shaft 14 passes through the lumen of the first shaft 12. Alternatively, only one shaft can be connected to one end of the protection sleeve while its other end is connected to the catheter, so the relative movement of the catheter and shaft can modify the protection sleeve shape.

In Fig. 1, protection sleeve 10 is in a contracted state, which is the state used to deliver the sleeve into a body lumen 16. "Contracted" refers to the radial direction about the first and second shafts 12 and 14. Protection sleeve 10 is elongate in the axial direction in this state. When in the axially elongated state, protection sleeve 10 adds minimal thickness to the contour of the shafts and catheter assembly. Fig. 1 shows the assembly delivered into vasculature of a patient. Protection sleeve 10 is positioned in the aortic arch near the carotid takeoffs 22. The pigtail distal portion 20 is positioned against one of the cusps of the aortic valve 24.

Reference is now made to Fig. 2. The first shaft 12 is slid distally over the second shaft 14, which is held stationary, which pushes sleeve 10 against the stationary junction of protection sleeve 10 with second shaft 14. This causes protection sleeve 10 to bunch up and expand radially outwards. It is noted that the axial length of protection sleeve 10 decreases during radial expansion of the sleeve. It is noted that in alternative embodiments of the invention, the first shaft can be the distal shaft and the second shaft can be the proximal shaft. It is also noted that protection sleeve 10 can alternatively be expanded by sliding second shaft 14 with respect to first shaft 12, which is held stationary.

In Fig. 2, the deployed protection sleeve 10 is deployed in the aortic arch and positioned to protect the carotid takeoffs 22 from emboli entering therein. The protection sleeve 10 diverts and traps potential calcific emboli, if such embolic debris are created during a surgical or catheteuzation procedure, such as but not limited to, valve repair or replacement surgery or catheterization. Emboli can flow into the distal axial end portion of sleeve 10 and flow out of the proximal axial end portion of sleeve 10. The middle section of sleeve 10 blocks emboli from entering the carotid takeoffs 22. Protection sleeve 10 does not impede or interfere with blood flow. After the procedure is completed, sleeve 10 can be retracted to its contracted state and removed from the lumen. It is noted that moving the relative positions of the two shafts with respect to the catheter can modify the position of the protection sleeve when in its deployed state. For example: the pigtail catheter can be delivered until its distal part is placed in the sinuses. The protection sleeve is then deployed by pushing the proximal shaft forward until the protection sleeve is fully open. Both shafts are now moved together, either forward or backwards until the protection sleeve is located to protect the carotid takeoffs, and the pigtail catheter is then pushed upwards so the protection sleeve "seals" the carotid takeoffs.

## Claims

1. A device comprising:
a protection sleeve (10) which has a proximal end joined to a first shaft (12) and a distal end joined to a second shaft (14), wherein said first shaft (12) slides over said second shaft (14);
a catheter (16) that passes through a lumen of said second shaft (14), wherein a distal portion (20) of said catheter (16) comprises a medical device; and wherein relative sliding movement of said first and second shafts (12, 14) with respect to each other either causes contraction or expansion of said protection sleeve (10).

2. The device according to claim 1, wherein an axial length of said protection sleeve (10) decreases during radial expansion of said protection sleeve (10).

3. The device according to claim 1, wherein distal sliding of said first shaft (12) over said second shaft (14), while said second shaft (14) is held stationary, pushes said protection sleeve (10) against a stationary junction of said protection sleeve (10) with said second shaft (14) and causes said protection sleeve (10) to expand radially outwards.

4. The device according to claim 1, wherein said catheter (16) comprises a pigtail catheter (16) with a pigtail at the distal portion (20).

5. The device according to claim 1, wherein said protection sleeve (10) is made of wire braid.

6. A device comprising:
a protection sleeve (10) which has a proximal end and a distal end;
a catheter (16) that passes through a lumen of a shaft (14), wherein a distal portion (20) of said catheter (16) comprises a medical device;
wherein one end of said protection sleeve (10) is connected to said catheter (16) and the other end is connected to said shaft (14) and wherein relative sliding movement of said catheter (16) and said shaft (14) with respect to each other either causes contraction or expansion of said protection sleeve (10).

## Patentansprüche

1. Vorrichtung, die Folgendes umfasst:
eine Schutzhülse (10), die ein proximales Ende, das mit einem ersten Schaft (12) verbunden ist, und ein distales Ende, das mit einem zweiten Schaft (14) verbunden ist, aufweist, wobei der erste Schaft (12) über den zweiten Schaft (14) gleitet;
einen Katheter (16), der ein Lumen des zweiten Schafts (14) passiert, wobei ein distaler Abschnitt (20) des Katheters (16) eine medizinische Vorrichtung umfasst; und wobei eine relative Gleitbewegung des ersten und des zweiten Schafts (12, 14) in Bezug aufeinander entweder eine Schrumpfung oder eine Ausdehnung der Schutzhülse (10) bewirkt.

2. Vorrichtung nach Anspruch 1, wobei eine axiale Länge der Schutzhülse (10) während der radialen Ausdehnung der Schutzhülse (10) abnimmt.

3. Vorrichtung nach Anspruch 1, wobei das distale Gleiten des ersten Schafts (12) über den zweiten Schaft (14), während der zweite Schaft (14) festgehalten wird, die Schutzhülse (10) gegen eine stationäre Verbindungsstelle zwischen der Schutzhülse (10) und dem zweiten Schaft (14) drückt und bewirkt, dass sich die Schutzhülse (10) radial nach außen hin ausdehnt.

4. Vorrichtung nach Anspruch 1, wobei der Katheter (16) einen Pigtail-Katheter (16) mit einem Pigtail am distalen Abschnitt (20) umfasst.

5. Vorrichtung nach Anspruch 1, wobei die Schutzhülse (10) aus einem Drahtgeflecht besteht.

6. Vorrichtung, die Folgendes umfasst:
eine Schutzhülse (10) mit einem proximalen Ende und einem distalen Ende;
einen Katheter (16), der ein Lumen eines Schafts (14) passiert, wobei ein distaler Abschnitt (20) des Katheters (16) eine medizinische Vorrichtung umfasst;
wobei ein Ende der Schutzhülse (10) mit dem Katheter (16) und das andere Ende mit dem Schaft (14) verbunden ist und wobei eine relative Gleitbewegung des Katheters (16) und des Schafts (14) in Bezug aufeinander entweder eine Schrumpfung oder eine Ausdehnung der Schutzhülse (10) bewirkt.

## Revendications

1. Dispositif comprenant:
une enveloppe de protection (10) ayant une extrémité proximale jointe à un premier arbre (12) ainsi qu'une extrémité distale jointe à un second arbre (14), dans lequel ledit premier arbre (12) glisse au-dessus dudit second arbre (14) ;
un cathéter (16) passant à travers un lumen dudit second arbre (14), dans lequel une partie distale (20) dudit cathéter (16) comprend un dispositif médical ; et dans lequel un mouvement de glissement relatif desdits premier et second arbres (12, 14) l'un par rapport à l'autre provoque la contraction ou l'extension de ladite enveloppe de protection (10).

2. Dispositif selon la revendication 1, dans lequel une longueur axiale de ladite enveloppe de protection (10) décroît au cours de l'extension radiale de ladite enveloppe de protection (10).

3. Dispositif selon la revendication 1, dans lequel le glissement distale dudit premier arbre (12) au-dessus dudit second arbre (14), tandis que ledit second arbre (14) est maintenu immobile, pousse ladite enveloppe de protection (10) contre un joint immobile de ladite enveloppe de protection (10) avec ledit second arbre (14) et provoque l'extension radiale vers l'extérieur de ladite enveloppe de protection (10).

4. Dispositif selon la revendication 1, dans lequel ledit cathéter (16) comprend un cathéter à spirale (16) avec une queue en tire-bouchon au niveau de la partie distale (20).

5. Dispositif selon la revendication 1, dans lequel ladite enveloppe de protection (10) est faite de fil torsadé.

6. Dispositif comprenant:
une enveloppe de protection (10) ayant une extrémité proximale et une extrémité distale;
un cathéter (16) passant à travers un lumen d'un arbre (14), dans lequel une partie distale (20) dudit cathéter (16) comprend un dispositif médical ;
dans lequel une extrémité de ladite enveloppe de protection (10) est reliée audit cathéter (16) et l'autre extrémité est relié audit arbre (14) et dans lequel le mouvement de glissement relatif dudit cathéter (16) et ledit arbre (14) l'un par rapport à l'autre provoque soit une contraction soit une extension de ladite enveloppe de protection (10).
